# Europäisches Patentamt
## European Patent Office
## Office européen des brevets

(19)

(11) Veröffentlichungsnummer: **0 219 784**
**B1**

(12) **EUROPÄISCHE PATENTSCHRIFT**

(45) Veröffentlichungstag der Patentschrift: **12.12.90**

(51) Int. Cl.⁵: **A 61 K 47/00**, A 61 K 9/00, A 61 K 31/495

(21) Anmeldenummer: **86114131.5**

(22) Anmeldetag: **13.10.86**

(54) **Infusionslösungen der 1-Cyclopropyl-6-fluor-1,4-dihydro-4-oxo-7-(1-piperazinyl)-chinolon-3-carbonsäure.**

(30) Priorität: **24.10.85 DE 3537761**

(43) Veröffentlichungstag der Anmeldung:
**29.04.87 Patentblatt 87/18**

(45) Bekanntmachung des Hinweises auf die Patenterteilung:
**12.12.90 Patentblatt 90/50**

(84) Benannte Vertragsstaaten:
**AT BE CH DE ES FR GB GR IT LI NL SE**

(56) Entgegenhaltungen:
EP-A-0 067 666
EP-A-0 138 018

**Die Akte enthält technische Angaben, die nach dem Eingang der Anmeldung eingereicht wurden und die nicht in dieser Patentschrift enthalten sind.**

(73) Patentinhaber: **BAYER AG**
**D-5090 Leverkusen 1 Bayerwerk (DE)**

(72) Erfinder: **Lammens, Robert, Dr.**
**Heymannstrasse 50**
**D-5090 Leverkusen 1 (DE)**
Erfinder: **Mahler, Hans, Dr.**
**Roggendorfstrasse 55**
**D-5000 Köln 80 (DE)**
Erfinder: **Serno, Peter, Dr.**
**An der Ruthen 3**
**D-5000 Köln 80 (DE)**

(56) Entgegenhaltungen:
**COMPTES RENDUES HEBDOMADAIRES DES SEANCES DE L'ACADEMIE DES SCIENCES; Band 292, Nr. 1, Serie III, 05.Jan. 1981, Seiten 37-40;Paris, FR. Y.GOUEFFON et al.: "Microbiologie - Sur un nouvel antibactérien de synthèse:l'acide éthyl-1 fluoro-6(méthyl-4 pipérazinyl-1)-7oxo-4 dihydro-1.4 quinoléine-3 carboxylique (1589 R.B.)."**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

(56) Entgegenhaltungen:
CHEMICAL ABSTRACTS, Band 103, Nr. 17,
28.Okt. 1985, Seite 19, Nr. 134433x; Columbus,
Ohio, US M.A. GONZALEZ et al.: "Multipledose
pharmacokinetics of ciprofloxacin administered
intravenously to normal volunteers"

CHEMICAL ABSTRACTS, Band 105, Nr. 11, 15.
Sept. 1986, Seite 10, Nr. 90716b; Columbus,
Ohio, US K. BORNER et al.: " Pharmacokinetics
of ciprofloxacin in healthy volunteers after oral
and intravenous administration".

Antimicrob Agents & Chemoth. 27, 375(1985)

CHEMICAL ABSTRACTS, Band 102, Nr. 21, 27.
Mai 1985, Seite 11, Nr. 178690n; Columbus,
Ohio, US G.HOEFFKEN et al.: "Pharmacokinetics
of ciprofloxacin after oral and parental
administration."

**Beschreibung**

Die Erfindung betrifft sowohl gebrauchsfertige Infusionslösungen der 1-Cyclopropyl-6-fluor-1,4-dihydro-4-oxo-7-(1-piperazinyl)-chinolin-3-carbonsäure (=Ciprofloxacin, im folgenden als Wirkstoff zu bezeichnen) wie auch sonstige Darreichungsformen, die vor der Applikation in solche Infusionslösungen überführt werden. Das Verfahren zur Herstellung der Infusionslösungen ist ebenfalls Gegenstand der Erfindung.

Lösungen milchsaurer Salze von Piperazinylchinolin- und Piperazinyl-azachinolincarbonsäuren werden in der europäischen Patentanmeldung EP—A—138018 beschrieben.

1-Cyclopropyl-6-fluor-1,4-dihydro-4-oxo-7-(1-piperazinyl)-chinolin-3-carbonsäure und deren pharmazeutisch verwendbare Salze sind Gegenstand der europäischen Patentanmeldung EP—A—49355.

In Antimicrobial Agents and Chemotherapy 27, 375 (1985) werden von Gert Höffken et al. wäßrige Ciprofloxacin-Lösungen beschrieben. Dazu muß festgestellt werden, daß eine Lösung von 50 mg oder 100 mg Ciprofloxacinlaktat mit einem Molverhältnis Ciprofloxacin/Milchsäure gleich 1 in 50 ml 0,9% NaCl-Lösung nicht herstellbar ist. In derartigen Zubereitungen geht der Wirkstoff nicht in Lösung.

Die vorliegende Erfindung betrifft Infusionslösungen der 1-Cyclopropyl-6-fluor-1,4-dihydro-4-oxo-7-(1-piperazinyl)-chinolin-3-carbonsäure (=Ciprofloxacin), enthaltend 0,015 bis 0,5 g des Wirkstoffs auf 100 ml wäßrige Lösung und je nach Wirkstoffkonzentration 1,04 bis 2,20 Mol, bezogen auf 1 Mol Wirkstoff, einer oder mehrerer physiologisch verträglicher Säuren, wobei für den Fall der Anwesenheit mehrerer Säuren deren Gesamtgehalt die Menge von 2,20 Mol, bezogen auf 1 Mol Wirkstoff, nicht übersteigt.

Bevorzugt enthalten die erfindungsgemäßen Infusionslösungen neben dem Wirkstoff, Wasser und weiterer üblichen Formulierungshilfsmitteln eine zur Lösung des Wirkstoffs und zur Stabilisierung der Lösung ausreichende Menge einer oder mehrerer Säure(n) aus der Gruppe bestehend aus Salzsäure, Methansulfonsäure, Propionsäure, Bernsteinsäure, Glutarsäure, Zitronensäure, Fumarsäure, Maleinsäure, Weinsäure, Glutaminsäure, Glukonsäure, Glukuronsäure, Galakturonsäure, Ascorbinsäure, Phosphorsäure, Adipinsäure, Hydroxyessigsäure, Schwefelsäure, Salpetersäure, Essigsäure, Apfelsäure, L-Asparaginsäure und Milchsäure.

Milchsäure und Salzsäure bzw. Gemische von Milchsäure und Salzsäure sind besonders bevorzugt.

Weiterhin bevorzugt sind Infusionslösungen, die 0,015 bis 0,5 g des Wirkstoffs auf 100 ml wäßrige Lösung und je nach Wirkstoffkonzentration bis zu 5,0 Mol, insbesondere 0,9 bis 5,0 Mol und besonders bevorzugt 1,04 bis 2,20 Mol, bezogen auf 1 Mol Wirkstoff, einer oder mehrerer physiologisch verträglicher Säuren enthalten, wobei für den Fall der Anwesenheit mehrerer Säuren deren Gesamtgehalt die Menge von 5,0 Mol, bezogen auf 1 Mol Wirkstoff, nicht übersteigt.

Bevorzugts betrifft die Erfindung auch Infusionslösungen, die 0,015 bis 0,5 g des Wirkstoffs auf 100 ml wäßrige Lösung und 1,04 bis 1,40 Mol, bezogen auf 1 Mol Wirkstoff, Milchsäure enthalten. Besonders vorteilhaft sind Infusionslösungen des Wirkstoffs, die 1,12 bis 1,24 Mol, bezogen auf 1 Mol Wirkstoff, Milchsäure enthalten.

Die erfindungsgemäßen Infusionslösungen können auch so modifiziert werden, daß sie bis zu 0,5 g des Wirkstoffs auf 100 ml wäßrige Lösung und bis zu 1 Mol, bezogen auf 1 Mol Wirkstoff, Milchsäure sowie eine weitere physiologisch verträgliche Säure enthalten, mit der Maßgabe, daß die gesamte Säuremenge je nach Wirkstoffkonzentration mehr als 1.04 Mol beträgt, jedoch 2,2 Mol, bezogen auf 1 Mol Wirkstoff, nicht übersteigt.

Die per Mol Wirkstoff zur Auflösung minimal notwendige Menge Säure hängt von der Wirkstoffkonzentration und der (den) verwendete(n) Säure(n) ab und ist somit nicht konstant. Sie kann jedoch in den Erfindungsgemäßen Grenzen durch einfache Versuche ermittelt werden. Weiterhin ist zu beachten, daß die Angaben in den Säuremengen sich nur auf die Mengen beziehen, die nach allgemein bekannten chemischen Grundregeln nicht durch Zugabe von Basen in das oder die entsprechende(n) Salz(e) umgesetzt werden. Eine Dissoziation der Säuren wurde bei den Mengenangaben außer Betracht gelassen, so daß sie sich auf die dissoziierte und undissoziierte Säuremenge beziehen.

Die in den Formulierungen eingesetzte Milchsäure hat einen Gehalt von weniger als 25% (g/g), und zwar aus verfahrenstechnischen Gründen. Verwendung von konzentrierter Milchsäure—z.B. 90 %ige (g/g) Ware—führt zu Schwierigkeiten, wenn nach Zugabe der Milchsäure der pH-Wert der erfindungsgemäßen Formulierungen eingestellt werden soll—z.B. mit Salzsäure oder Natronlauge—mit dem Ziel, daß der eingestellte pH-Wert im Laufe des restlichen Herstellverfahrens (wie z.B. eine Hitzebehandlung bei ca. 120°C während ca. 20 min) und/oder der Lagerung konstant bleibt oder sich nur noch unwesentlich verändert.

Die erfindungsgemäßen Infusionslösungen können auch weitere Formulierungsmittel wie Verdickungsmittel, Resorptionsmittel, Lichtschutzmittel, Resorptionshemmer, Kristallisationsbeschleuniger, Resorptionsbeschleuniger, Kristallisationsverzögerer, Komplexierungsmittel, Antioxidantia, Isotonisierungsmittel und/oder Euhydrierungsmittel enthalten.

Die Osmolalität der Infusionslösungen beträgt 0,20 bis 0,70 Osm/kg, bevorzugt 0,26 bis 0,39 Osm/kg und wird durch Isotonisierungsmittel wie NaCl, Sorbit, Mannit, Glukose, Saccharose, Xylit, Fruktose und Glycerin oder Gemische solcher Substanzen eingestellt. Gegebenenfalls können dazu auch Substanzen eingesetzt werden, die in gängigen, im Handel erhältlichen Infusionsträgerlösungen enthalten sind.

Zu den üblichen Infusionsträgerlösungen gehören Infusionslösungen mit Elektrolytzufuhr ohne Kohlehydrate wie Kochsalzlösung, Ringer-Lactat-Lösung und ähnliche und solche mit Kohlehydraten sowie Lösungen zur Zufuhr von Aminosäuren, jeweils mit und ohne Kohlehydratanteil. Beispiele für solche Infusionsträgerlösungen sind in Rote Liste 1985, Verzeichnis von Fertigarzneimitteln der Mitglieder des Bundesverbandes der Pharmazeutischen Industrie e.V., Editio Cantor, Aulendorf/Württ. aufgeführt.

Bevorzugt sind Infusionslösungen die außer Wasser, Wirkstoff und sonstigen Formulierungshilfsmitteln eine derartige Menge Kochsalz oder anderer zur Isotonisierung übliche Hilfsstoffe enthalten, daß eine mit der Gewebeflüssigkeit des menschlichen oder tierischen Körpers isotone oder geringfügig hypo- oder hypertone Lösung vorliegt.

Die Infusionslösungen gemäß der Erfindung weisen einen pH-Wert von 3,0 bis 5,2 auf. pH-Werte von 3,6 bis 4,7 bzw. 3,9 bis 4,5 sind bevorzugt. Ganz besonders bevorzugt sind pH-Werte im Bereich von 4,1 bis 4,3.

Eine ganz besonders bevorzugte Ausführungsform der Erfindung sind Infusionslösungen, die außer Wirkstoff, Wasser und sonstigen Formulierungshilfsmitteln je nach Wirkstoffmenge 1,04 bis 1,40 Mol Milchsäure und 0,0 0 bis 0,80 Mol Salzsäure (jeweils bezogen auf 1 Mol Wirkstoff) und bezogen auf 100 ml Lösung 0,6 bis 2,2 g NaCl, vorzugsweise 0,75 bis 1,20 g, insbesondere 0,85 bis 0,95 g NaCl, enthalten. Die so erhaltenen Lösungen besitzen je nach Kochsalzmenge und Wirkstoffkonzentration unterschiedliche Osmolalitäten. Die zu den oben aufgeführten Kochsalzmengen gehörenden Osmolalitäten betragen 0,2 bis 0,7, bzw. 0,26 bis 0,39 und 0,28 bis 0,32 Osm/kg Lösung. Entsprechende Werte können auch mit anderen Isotonisierungsmitteln oder Gemischen derselben wie oben angegeben eingestellt werden. Je nach Wirkstoff und Säurekonzentration sind geringe Abweichungen von diesen Osmolalitäten durchaus möglich.

Die erfindungsgemäßen Infusionslösungen können in zur Infusion geeigneten Dosierungseinheiten mit entnehmbaren Inhalten von 40 bis 600 ml, vorzugsweise 50 bis 120 ml vorliegen.

Jedoch gehören auch nach üblichen Techniken hergestellte Lyophilisate, die durch Auflösen in dazu geeigneten Lösungsmitteln—wie z.B. gänge Infusionsträgerlösungen—in die erfindungsgemäßen Infusionslösungen überführt werden, zum Gegenstand der Erfindung. Solche Lyophilisate können erhalten werden durch Gefriertrocknung verschiedenster Ausgangslösungen, wie z.B. die erfindungsgemäßen Infusionslösungen. Ebenfalls können wesentlich stärker verdünnte Lösungen wie auch wesentlich konzentriertere Lösungen als die erfindungsgemäßen Infusionslösungen gefriergetrocknet werden.

Die Lyophilisate können sowohl durch eine Gefriertrocknung in dem Endbehälter wie z.B. in einer Flasche oder Ampulle aus Glas oder Kunststoff als auch durch eine Bulkgefriertrocknung, verbunden mit einer zu einem späteren Zeitpunkt stattfindenden Abfüllung des Lyophilisats in ein dazu geeignetes Behältnis, hergestellt werden.

Das Auflösen des Lyophilisats vor der Applikation kann sowohl durch Zugabe einer dazu geeigneten Lösung in das das Lyophilisat enthaltende Behältnis geschenen, wie auch durch Zugabe des Lyophilisats zu einer geeigneten Lösung, oder durch eine Kombination solcher Vorgehensweisen.

Die Zusammensetzung der Lyophilisate kann ebenfalls sehr verschieden sein, je nach Zusammensetzung der Lösung, die zum Auflösen benutzt wird.

Sie kann variieren von reinem Wirkstoff bis zu einem Lyophilisat, das bis auf Wasser sämtliche zu applizierende Bestandteile enthält.

Kombinationen von Lyophilisaten mit wirkstoffhaltigen Lösungen, die vor der Applikation in die erfindungsgemäßen Infusionslösungen überführt werden, sind ebenfalls Gegenstand der Erfindung.

Außerdem gehören Konzentrate und Suspensionen, die vor der Applikation in die erfindungsgemäßen Lösungen überführt werden, zur Erfindung.

Dabei können diese Konzentrate und Suspensionen verschiedene Zusammensetzungen haben. Eine Möglichkeit wäre die, die nur noch die Zugabe von Wasser zur Verdünnung bzw. Auflösung erfordert, um die erfindungsgemäßen Infusionslösungen herzustellen.

Sämtliche Kombinationen von Konzentraten und/oder Suspensionen und zur Verdünnung bzw. Auflösung erforderlichen Lösungen, die zu den erfindungsgemäßen Lösungen führen, sind Gegenstand dieser Erfindung.

Auch sonstige Darreichungsformen oder Kombinationen von Darreichungsformen, die letztendlich zu den erfindungsgemäßen Infusionslösungen führen—und dies unabhängig von der Vorgehensweise—sind Gegenstand dieser Erfindung.

Die Behältnisse, in die Lyophilisate, Konzentrate und sonstige Darreichungsformen wie z.B. Suspensionen abgefüllt werden, können sowohl aus Glas wie auch aus Kunststoff bestehen. Dabei können die Behältermaterialien Substanzen enthalten, die dem Inhalt einen besonderen Schutz verleihen, wie z.B. einen Lichtschutz oder einen Sauerstoffschutz.

Die vorliegende Erfindung betrifft außerdem ein Verfahren zur Herstellung von Infusionslösungen, enthaltend 0,015 bis 0,5 Gew.-% 1-Cyclopropyl-6-fluor-1,4-dihydro-4-oxo-7-(1-piperazinyl)-chinolin-3-carbonsäure (=Ciprofloxacin). Dieses Verfahren besteht darin, daß man eine geeignete Menge des Wirkstoffs, gegebenenfalls in Form eines Salzes wie Alkali- oder Erdalkalisalz oder Additionssalzes, eines Hydrates oder eines Hydrates des Salzes oder in Form von Mischungen dieser Salze bzw. Hydrate, mit der Menge einer physiologisch verträglichen Säure versetzt oder eines Gemisches mehrerer physiologisch verträglicher Säuren, die in bezug auf die eben auf Auflösung des Wirkstoffs bzw. seiner Salze oder Hydrate ausreichende Menge einen Ausscheidungen des Wirkstoffs verhindernden Überschuß darstellt, gegebenenfalls Formulierungshilfsmittel hinzufügt und mit Wasser oder einer üblichen Infusionsträgerlösung derart ausfüllt, daß sich ein Konzentrationsbereich von 0,015 bis 0,5 g für den Wirkstoff einstellt. Dabei ist zu bedenken, daß beim Einsetzen der Alkali- oder Erdalkalisalze des Wirkstoffes in den oben erwähnten zur Auflösung erforderlichen Säuremengen die Menge enthalten ist, die zur Neutralisation des Wirkstoffanions notwendig ist und daß bei dem Einsatz von Additionssalzen ein Teil der erforderlichen Säuremengen bereits im einzusetzenden Wirkstoffsalz enthalten ist.

Bei der Herstellung ist weiterhin zu beachten, daß die Lösung den bereits aufgeführten Eigenschaften hinsichtlich pH, Säuremengen und Osmolalitäten entspricht.

Für den Fall der Verwendung des Wirkstoffs in Salzform kann zweckmäßig eine Säure eingesetzt werden, deren Anion dem Anion des Wirkstoffsalzes bzw. des -salz-hydrates entspricht.

Gegebenenfalls suspendiert man den Wirkstoff in Wasser, versetzt mit bis zu 5 Mol, bezogen auf 1 Mol Wirkstoff.

Milchsäure und fügt dann gegebenenfalls eine weitere physiologisch verträgliche Säure oder ein Gemisch solcher Säuren, insbesondere Salzsäure, hinzu, mit der Maßgabe, daß die Gesamtmenge an Säure 5,0 Mol, bezogen auf 1 Mol Wirkstoff, nicht überschreitet, jedoch 0,9 Mol, bezogen auf 1 Mol Wirkstoff, übersteigt und gibt anschließend gegebenenfalls weitere Formulierungshilfsmittel, insbesondere NaCl, das gegebenenfalls auch durch eine Neutralisationsreaktion im Formulierungsansatz entsteht, hinzu und füllt mit Wasser auf zur Einstellung der gewünschten Wirkstoffkonzentration.

Der pH-Wert der erfindungsgemäßen Infusionslösungen läßt sich mit (physiologisch) verträglichen Säuren und/oder Basen auf die obengenannten Werte, d.h. 3,0 bis 5,2, insbesondere 3,6 bis 4,7 einstellen.

Zur Beschleunigung des Herstellverfahrens, insbesondere des Auflösens von festen Komponenten können die Lösungen oder nur ein Teil davon geringfügig erwärmt werden, vorzugsweise auf Temperaturen zwischen 20°C und 80°C.

Besonders wirtschaftlich konnten die erfindungsgemäßen Lösungen hergestellt werden über konzentrierte Lösungen. Dazu wurde die für einen Ansatz erforderliche Menge an Wirkstoff mit der für den kompletten Ansatz erforderlichen Hauptmenge an Säure (z.B.) 95%, bezogen auf Molbasis) in ein wenig Wasser—gegebenenfalls unter Erwärmen—gelöst. Dieses Konzentrat wurde dann anschließend verdünnt. Nach Verdünnung wurden die eventuellen sonstigen Hilfsstoffe—wie z.B. Kochsalz zur Isotonisierungzugegeben, und ebenso die gegebenenfalls noch fehlenden Säuremengen.

Die erfindungsgemäßen Infusionslösungen finden Verwendung bei der therapeutischen Behandlung des menschlichen oder tierischen Körpers.

Die erfindungsgemäßen Infusionslösungen zeigen bei geringer Toxizität ein breites antibakterielles Spektrum gegen gram-positive und gram-negative Keime, insbesondere gegen Enterobakteriaceen; vor allem auch gegen solche, die resistent sind gegen verschiedene Antibiotika, wie z.B. Penicilline, Cephalosporine, Aminoglykoside, Sulfonamide, Tetracycline.

Diese wertvollen Eigenschaften ermöglichen ihre Verwendung in der Medizin.

Die erfindungsgemäßen Infusionslösungen sind gegen ein sehr breites Spektrum von Mikroorganismen wirksam. Mit ihrer Hilfe können gram-negative und gram-positive Bakterien und bakterienähnliche Mikroorganismen bekämpft sowie die durch diese Erreger hervorgerufenen Erkrankungen verhindert, gebessert und/oder geheilt werden.

Besonders wirksam sind die erfindungsgemäßen Infusionslösungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Beispielsweise können lokale und/oder systemische Erkrankungen behandelt und/oder verhindert werden, die durch die folgenden Erreger oder durch Mischungen der folgenden Erreger verursacht werden:

Gram-positive Kokken, z.B. Staphylokokken (Staph. aureus, Staph. epidermidis) und Streptokokken (Strept. agalactiae, Strept. faecalis, Strept. pneumoniae, Strept. pyogenes); gram-negative Kokken (Neisseria gonorrhoeae) sowie gram-negative Stäbchen wie Enterobackteriaceen, z.B. Escherichia coli, Hämophilus influenzae, Citrobacter (Citrob. freundii, Citrob. divernis), Salmonella und Shigella; ferner Klebsiellen (Klebs. pneumoniae, Klebs. oxytoca), Enterobacter (Ent. aerogenes, Ent. agglomerans), Hafnia, Serratia (Serr. marcescens), Proteus (Pr. mirabilis, Pr. rettgeri, Pr. vulgaris), Providencia, Yersinia, sowie die Gattung Acinetobacter. Darüber hinaus umfaßt das antibackterielle Spektrum die Gattung Pseudomonas (Ps. auruginosa, Ps. maltophilia) sowie strikt anaerobe Baktieren wie z.B. Bacteroides fragilis, Vertreter der Gattung Peptococcus, Peptostreptococcus sowie die Gattung Clostridium; ferner Mykoplasmen (M. pneumoniae, M. hominis, M. urealyticum) sowie Mykobaktieren, z.B. Mycobacterium tuberculosis.

Die obige Aufzählung von Erregern ist lediglich beispielhaft und keineswegs beschränkend aufzufassen. Als Krankheiten, die durch die genannten Erreger oder Mischinfektionen verursacht und durch die erfindungsgemäßen Verbindungen verhindert, gebessert oder geheilt werden können, seinen beispielsweise genannt:

Infektionskrankheiten beim Menschen wie zum Beispiel Otitis, Pharyngitis, Pneumonie, Peritonitis, Pyelonephritis, Cystitis, Endocarditis, Systeminfektionen, Bronchitis (akut, chronisch), septische Infektionen, Erkrankungen der oberen Luftwege, diffuse Panbronchiolitis, pulmonäres Emphysem, Dysenterie, Enteritis, Leberabszesse, Urethritis, Prostatitis, Epididymitis, gastrointestinale Infektionen, Knochen- und Gelenkinfektionen, zystische Fibrose, Hautinfektionen, postoperative Wundinfektionen, Abszesse, Phlegmone, Wundinfektionen, infizierte Verbrennungen, Brandwunden, Infektionen im Mundbereich, Infektionen nach Zahnoperationen, Osteomyelitis, septische Arthritis, Cholecystitis, Peritonitis mit Appendicitis, Cholangitis, intraabdominale Abszesse, Pankreatitis, Sinusitis, Mastoiditis, Mastitis, Tonsillitis, Typhus, Meningitis und Infektionen des Nervensystems, Salpingitis, Endometritis, Genital-Infektionen, Pelveoperitonitis und Augeninfektionen.

Außer beim Menschen können bakterielle Infektionen auch bei anderen Spezies behandelt werden. Beispielhaft seien genannt:

Schwein: Coli-diarrhoe, Enterotoxämie, Sepsis, Dysenterie, Salmonellose, Metritis-Mastitis-Agalaktiae-Syndrom, Mastitis;

Wiederkäuer (Rind, Schaf, Ziege): Diarrhoe, Sepsis, Bronchopneumonie, Salmonellose, Pasteuerellose, Mykoplasmose, Genitalinfektionen;

Pferd: Bronchopneumonien, Fohlenlähme, puerperale und postpuerperale Infektionen, Salmonellose;

Hund und Katze: Bronchopneumonie, Diarrhoe, Dermatitis, Otitis, Harnwegsinfekte, Prostatitis;

Geflügel (Huhn, Pute, Wachtel, Taube, Ziervögel und andere): Mycoplasmose, E. coli-Infektionen, chronische Luftwegserkrankungen, Salmonellose, Pasteuerellose, Psittakose.

Ebenso können bakterielle Erkrankungen bei der Aufzucht und Haltung von Nutz- und Zierfischen behandelt werden, wobei sich das antibakterielle Spektrum über die vorher genannten Erreger hinaus auf weitere Erreger wie zum Beispiel Pasteurella, Brucella, Camphylobacter, Listeria, Erysipelothrix, Corynebakterien, Boreillia, Treponema, Nocardia, Rikettsien, Yersinia, erweitert.

Beispiele

Das in den nachstehenden Beispielen aufgeführte Molverhältnis—abgekürzt mit R—bezieht sich immer auf die in dem betroffenen Beispiel erstgenannte Substanz.

| Beisp. Nr. | Formulierung | | Mikromol | R |
|---|---|---|---|---|
| 1 | Ciprofloxacin | 90 mg | 272 | 1.00 |
| | Milchsäure 20% (g/g) | 144,3 mg | 320 | 1.18 |
| | Salzsäure | 1,5 mg | 48 | 0.15 |
| | Kochsalz | 5,4 g | — | — |
| | Wasser | ad 600,0 ml | — | — |
| | | pH: ca. 4,3 | | |
| | | Osm: ca. 0,29 Osm/kg | | |
| 2 | Ciprofloxacin | 150 mg | 453 | 1.00 |
| | Milchsäure 10% (g/g) | 558 mg | 658 | 1.37 |
| | Salzsäure | 7,8 mg | 214 | 0.47 |
| | Glukose | 30,0 mg | — | — |
| | Wasser | ad 600,0 ml | — | — |
| | | pH: ca. 3,7 | | |
| | | Osm: ca. 0,29 Osm/kg | | |
| 3 | Ciprofloxacinacetat | 118 mg | 302 | 1.00 (darin enthalten equimolare Menge Essigsäure) |
| | Essigsäure | 3,6 mg | 60 | 0.20 |
| | Milchsäure 10% (g/g) | 272 mg | 302 | 1.00 |
| | Sorbit | 10,0 g | — | — |
| | Wasser | ad 200,0 ml | — | — |
| | | pH: 4,7 | | |
| | | Osm: 0,30 Osm/kg | | |
| 4 | Ciprofloxacin | 200 mg | 604 | 1.00 |
| | Propionsäure | 54 mg | 724 | 1.20 |
| | Milchsäure 20% g/g | 136 mg | 604 | 1.00 |
| | Glukose | 20,0 g | — | — |
| | Wasser | ad 400,0 ml | — | — |
| | | pH: 4,7 | | |
| | | Osm: 0,3 Osm/kg | | |

6

TABLE (continued)

| Beisp. Nr. | Formulierung | | Mikromol | R |
|---|---|---|---|---|
| 5 | Ciprofloxacin | 200 mg | 604 | 1.00 |
| | Milchsäure 1% (g/g) | 7440 mg | 826 | 1.37 |
| | 1N Salzsäure | 0,285 ml | 285 | 0.47 |
| | Mannit | 6,2 g | — | — |
| | Wasser | ad 200 ml | | |
| | | pH: 3,7 | | |
| | | Osm: 0,37 Osm/kg | | |
| 6 | Ciprofloxacin | 50 mg | 151 | 1.00 |
| | Milchsäure 10% (g/g) | 160 mg | 178 | 1.18 |
| | Kochsalz | 625 mg | | |
| | Wasser | ad 50 ml | | |
| | | pH: 4,4 | | |
| | | Osm: 0,40 Osm/kg | | |
| 7 | Ciprofloxacin | 500 mg | 1509 | 1.00 |
| | Milchsäure 2% (g/g) | 6727 mg | 1494 | 0.99 |
| | Sorbit | 25,0 g | | |
| | Wasser | ad 500 ml | | |
| | | pH: 5,0 | | |
| | | Osm: 0,29 Osm/kg | | |
| 8 | Ciprofloxacinlaktat | 127 mg | 302 | 1.00 (darin enthalten equimolare Menge Milchsäure) |
| | Maleinsäure | 42 mg | 364 | 1.20 |
| | Mannit | 5,0 g | | |
| | Wasser | ad 100,0 ml | | |
| | | pH: 3,1 | | |
| | | Osm: 0,30 Osm/kg | | |
| 9 | Ciprofloxacin | 200 mg | 604 | 1.00 |
| | Milchsäure 20% (g/g) | 297 mg | 659 | 1.09 |
| | Glutarsäure | 40 mg | 302 | 0.50 |
| | Fruktose | 10,0 g | | |
| | Wasser | ad 200 ml | | |
| | | pH: 4,3 | | |
| | | Osm: 0,30 Osm/kg | | |
| 10 | Ciprofloxacin | 200 mg | 604 | 1.00 |
| | Milchsäure 10% (g/g) | 566 mg | 628 | 1.04 |
| | Kochsalz | 0,9 g | — | — |
| | Wasser | ad 100,0 ml | — | — |
| | | pH: 4,9 | | |
| | | Osm: 0,29 Osm/kg | | |
| 11 | Ciprofloxacin | 400 mg | 1207 | 1.00 |
| | Milchsäure 20% (g/g) | 745 mg | 1654 | 1.37 |
| | Salzsäure | 20,8 mg | 570 | 0.47 |
| | Kochsalz | 1800 mg | | |
| | Wasser | ad 200 ml | | |
| | | pH: 3,7 | | |
| | | Osm: 0,29 Osm/kg | | |

| Beisp. Nr. | Formulierung | | Mikromol | R |
|---|---|---|---|---|
| 12 | Ciprofloxacin · 5 $H_2O$ | 509 mg | 1207 | 1.00 |
| | Milchsäure 5% (g/g) | 2568 mg | 1425 | 1.18 |
| | Salzsäure | 6,6 mg | 180 | 0.15 |
| | Glukose | 5,0 g | — | — |
| | Wasser | ad 200 ml | — | — |
| | | pH: 4,2 | | |
| | | Osm: 0,30 Osm/kg | | |
| 13 | Ciprofloxacin | 100 mg | 302 | 1.00 |
| | Milchsäure 0,4% (g/g) | 7407 mg | 329 | 1.09 |
| | 1 m Salzsäure | 3,42 ml | 3420 | 11.32 |
| | 2 m Natronlauge | 1,71 ml | 3420 | 11.32 |
| | Kochsalz | 0,25 g | | |
| | Wasser | ad 50,0 ml | | |
| | | pH: 4,7 | | |
| | | Osm: Osm/kg | | |
| 14 | Ciprofloxacin HCl · $H_2O$ | 232 mg | 604 | 1.00 (darin enthalten equimolare Menge Salzsäure) |
| | Milchsäure 5% (g/g) | 1288 mg | 715 | 1.18 |
| | 2 m Natronlauge | 0.257 ml | 514 | 0.85 |
| | Kochsalz | 0,870 g | | |
| | Wasser | ad 100 ml | | |
| | | pH: 4,2 | | |
| | | Osm: 0,30 Osm/kg | | |
| 15 | Ciprofloxacin Na (Natriumsalz von Ciprofloxacin) | 213 mg | 604 | 1.00 |
| | Milchsäure 10% (g/g) | 744 mg | 826 | 1.37 |
| | Salzsäure | 32,4 mg | 888 | 1.47 |
| | Glukose | 4,8 g | | |
| | Wasser | ad 100,0 ml | | |
| | | pH: 3,7 | | |
| | | Osm: 0,30 Osm/kg | | |
| 16 | Calciumsalz von Ciprofloxacin | 212 mg | 604 | 1.00 |
| | Milchsäure 2% (g/g) | 3220 mg | 715 | 1.18 |
| | Salzsäure | 25,3 mg | 693 | 1.15 |
| | Glycerin | 2,6 g | — | — |
| | Wasser | ad 500,0 ml | | |
| | | pH: 4,3 | | |
| | | Osm: 0,30 Osm/kg | | |
| 17 | Kaliumsalz von Ciprofloxacin | 233 mg | 604 | 1.00 |
| | Milchsäure 20% (g/g) | 277 mg | 615 | 1.018 |
| | 0,1 m Salzsäure | 8,86 ml | 886 | 1.47 |
| | Glukose | 5,0 g | | |
| | Wasser | ad 100,0 ml | | |
| | | pH: 4,6 | | |
| | | Osm: 0,30 Osm/kg | | |

# EP 0 219 784 B1

| Beisp. Nr. | Formulierung | | Mikromol | R |
|---|---|---|---|---|
| 18 | Ciprofloxacinglukonat | 159 mg | 302 | 1.00 (darin enthalten equimolare Menge Glukonsäure) |
| | Glukonsäure | 12 mg | 62 | 0.20 |
| | Milchsäure 5% (g/g) | 544 mg | 302 | 1.00 |
| | Glycerin | 1,3 g | — | — |
| | Wasser | ad 50,0 ml | | |
| | | pH: 4,0 | | |
| | | Osm: 0,30 Osm/kg | | |
| 19 | Ciprofloxacin | 500 mg | 1509 | 1.00 |
| | Galacturonsäure | 351 mg | 1811 | 1.20 |
| | Sorbit | 25,0 g | | |
| | Wasser | ad 250,0 ml | | |
| | | pH: 4,6 | | |
| | | Osm: 0,3 Osm/kg | | |
| 20 | Ciprofloxacin · 5 $H_2O$ | 127 mg | 302 | 1.00 |
| | Ascorbinsäure | 41 mg | 366 | 1.21 |
| | Glukose | 2,5 g | | |
| | Wasser | ad 50 ml | | |
| | | pH: 4,5 | | |
| | | Osm: 0,30 Osm/kg | | |
| 21 | Ciprofloxacinlaktat | 509 mg | 1207 | 1.00 (darin enthalten equimolare Menge Milchsäure) |
| | Hydroxyessigsäure | 110 mg | 1448 | 1.20 |
| | Kochsalz | 1,8 g | | |
| | Wasser | ad 200 ml | | |
| | | pH: 4,0 | | |
| | | Osm: 0,30 Osm/kg | | |
| 22 | Ciprofloxacin | 100 mg | 302 | 1.00 |
| | L · asparaginsäure | 48 mg | 361 | 1.20 |
| | Fruktose | 2,5 g | | |
| | Wasser | ad 50,0 ml | | |
| | | pH: 4,5 | | |
| | | Osm: 0,30 Osm/kg | | |
| 23 | Ciprofloxacin | 100 mg | 302 | 1.00 |
| | Milchsäure 20% (g/g) | 204 mg | 453 | 1.50 |
| | 0,1 m Salzsäure | 2,11 ml | 211 | 0.70 |
| | Sorbit | 2,5 g | | |
| | Wasser | ad 50,0 ml | | |
| | | pH: 3,3 | | |
| | | Osm: 0,30 Osm/kg | | |
| 24 | Ciprofloxacin | 200 mg | 604 | 1.00 |
| | Milchsäure 15% (g/g) | 496 mg | 826 | 1.37 |
| | 0,2 m Salzsäure | 1,42 ml | 284 | 0.47 |
| | Glukose | 5,00 g | — | — |
| | Wasser | ad 40,0 ml | | |
| | | pH: 3,7 | | |
| | | Osm: 0,33 Osm/kg | | |

| Beisp. Nr. | Formulierung | | Mikromol | R |
|---|---|---|---|---|
| 25 | Ciprofloxacinlaktat · 2 H$_2$O | 3858 mg | 7544 | 1.00 (darin enthalten equimolare Menge Milchsäure) |
| | Kochsalz | 4,5 g | — | — |
| | 1 m Salzsäure | 0,754 ml | 754 | 0.10 |
| | Wasser | ad 500 ml | | |
| | | pH: 4,7 | | |
| | | Osm: 0,30 Osm/kg | | |
| 26 | Ciprofloxacin | 500 mg | 1509 | 1.00 |
| | Milchsäure 1% | 14814 mg | 1645 | 1.09 |
| | Kochsalz | 0,45 g | | |
| | Wasser | ad 100,0 ml | | |
| | | pH: 4,7 | | |
| | | Osm: 0,31 Osm/kg | | |
| 27 | Ciprofloxacinlaktat · 2 H$_2$O | 359 mg | 754 | 1.00 (darin enthalten equimolare Menge Milchsäure) |
| | Milchsäure 20% (g/g) | 2,6 mg | 69 | 0.09 |
| | Bernsteinsäure | 45 mg | 381 | 0.51 |
| | Sorbit | 2,5 g | | |
| | Wasser | ad 50,0 ml | | |
| | | pH: 4,2 | | |
| | | Osm: 0,32 Osm/kg | | |
| 28 | Ciprofloxacin | 500 mg | 1509 | 1.00 |
| | Milchsäure 10% (g/g) | 1481 mg | 1644 | 1.09 |
| | Zitronensäure | 159 mg | 757 | 0.50 |
| | Glukose | 5,0 g | | |
| | Wasser | ad 100,0 ml | | |
| | | pH: 3,8 | | |
| | | Osm: 0,33 Osm/kg | | |
| 29 | Ciprofloxacinlaktat | 127 mg | 302 | 1.00 (darin enthalten equimolare Menge Milchsäure) |
| | Ciprofloxacinfumarat | 68 mg | 151 | 0.50 darin enthaltene äquimolare Menge Fumar- säure |
| | Ciprofloxacin | 50 mg | 151 | 0.50 |
| | Milchsäure 20% | 160 mg | 356 | 1.18 |
| | Fruktose | 2,0 g | | |
| | Wasser | ad 40,0 ml | | |
| | | pH: 3,9 | | |
| | | Osm: 0,32 Osm/kg | | |
| 30 | Ciprofloxacin | 500 mg | 1509 | 1.00 |
| | Milchsäure 25% | 641 mg | 1780 | 1.18 |
| | Kochsalz | 0,9 g | | |
| | Wasser | ad 100,0 ml | | |
| | | pH: 4,5 | | |
| | | Osm: 0,30 Osm/kg | | |

| Beisp. Nr. | Formulierung | | Mikromol | R |
|---|---|---|---|---|
| 31 | Lösung A | | | |
| | Ciprofloxacin | 100 mg | 302 | 1.00 |
| | Milchsäure 6% (g/g) | 537 mg | 358 | 1.19 |
| | 0,1 m Salzsäure | 0,45 ml | 45 | 0.15 |
| | Kochsalz | 0,45 g | | |
| | Wasser | ad 50,0 ml | | |
| | | | | |
| | Lösung B | | | |
| | Glukose | 12,5 g | | |
| | Wasser | ad 250 ml | | |
| | Gebrauchsfertige Lösung herstellen durch Mischen von Lösung A und B | | | |
| 32 | Lösung A | | | |
| | Ciprofloxacin | 100 mg | 302 | 1.00 |
| | Milchsäure 2% (g/g) | 1610 mg | 358 | 1.19 |
| | Salzsäure | 1,6 mg | 45 | |
| | Glukose | 2,5 g | | |
| | Wasser | ad 50 ml | | |
| | | | | |
| | Lösung B | | | |
| | Kochsalz | 900 mg | | |
| | Wasser | ad 100 ml | | |
| | Gebrauchsfertige Lösung herstellen durch Mischen von Lösung A und B | | | |
| 33 | Lösung A | | | |
| | Ciprofloxacinlaktat · 2 $H_2O$ | 276 mg | 604 | 1.00 (darin enthalten equimolare Menge Milchsäure) |
| | Milchsäure 0,1% (g/g) | 10,0 g | 111 | 0.18 |
| | Salzsäure | 3,3 mg | 90 | 0.15 |
| | Xylit | 4,2 g | | |
| | Wasser | ad 100,0 ml | | |
| | | | | |
| | Lösung B | | | |
| | Fruktose | 10,0 g | | |
| | Wasser | ad 100,0 ml | | |
| | Gebrauchsfertige Lösung herstellen durch Mischen von Lösng A und B | | | |
| 34 | Lösung A | | | |
| | Ciprofloxacin | 200 mg | 604 | 1.00 |
| | Milchsäure 10% (g/g) | 644 mg | 715 | 1.18 |
| | 0,1 m Salzsäure | 0,90 ml | 90 | 0.15 |
| | Kochsalz | 0,9 g | | |
| | Wasser | ad 100,0 ml | | |
| | | | | |
| | Lösung B | | | |
| | Xylit | 12,5 g | | |
| | Wasser | ad 250 ml | | |
| | Gebrauchsfertige Lösung herstellen durch Mischen von Lösung A und B | | | |

| Beisp. Nr. | Formulierung | | Mikromol | R |
|---|---|---|---|---|
| 35 | Lösung A | | | |
| | Ciprofloxacin | 200 mg | 604 | 1.00 |
| | Milchsäure 1,5% (g/g) | 4960 mg | 826 | 1.37 |
| | 0,1 m Salzsäure | 2,84 ml | 284 | 0.47 |
| | Glukose | 5,00 g | | |
| | Wasser | ad 40,0 ml | | |
| | | | | |
| | Lösung B | | | |
| | Glukose | 5,0 g | | |
| | Wasser | ad 100 ml | | |
| | Gebrauchsfertige Lösung herstellen durch Mischen von Lösung A und B | | | |
| 36 | Lösung A | | | |
| | Ciprofloxacin | 100 mg | 302 | 1.00 |
| | Milchsäure 10% (g/g) | 372 mg | 413 | 1.37 |
| | Salzsäure | 5,2 mg | 142 | 0.47 |
| | Wasser | ad 10,0 ml | | |
| | | | | |
| | Lösung B | | | |
| | Kochsalz | 0,45 g | | |
| | Wasser | ad 50,0 ml | | |
| | Gebrauchsfertige Lösung herstellen durch Mischen von Lösung A und B | | | |
| 37 | Lösung A | | | |
| | Lösung A aus Beispiel 65 | | | |
| | | | | |
| | Lösung B | | | |
| | Fruktose | 5,0 g | | |
| | Wasser | ad 100,0 ml | | |
| | Gebrauchsfertige Lösung herstellen durch Mischen von Lösung A und B | | | |
| 38 | Lösung A | | | |
| | Lösung A aus Beispiel 65 | | | |
| | | | | |
| | Lösung B | | | |
| | Ringer Laktat-Lösung: | 250 ml | (z.B. Ringer Laktat DAB 7 Hersteller Fa. Braun Melsungen Rote Liste 1985 Nr. 51013) | |
| | Gebrauchsfertige Lösung herstellen durch Mischen von Lösung A und B | | | |
| 39 | Lösung A | | | |
| | Lösung A aus Beispiel 65 | | | |
| | | | | |
| | Lösung B | | | |
| | Mannit | 25,0 g | | |
| | Wasser | ad 500,0 ml | | |
| | Gebrauchsfertige Lösung herstellen durch Mischen von Lösung A und B | | | |
| 40 | Lösung A | | | |
| | Ciprofloxacin | 250 mg | 754 | 1.00 |
| | Milchsäure 20% (g/g) | 465 mg | 1032 | 1.37 |
| | 0,1 m Salzsäurelösung | 1,63 ml | 163 | 0.22 |
| | Kochsalz | 173 mg | | |
| | Wasser | ad 25 ml | | |
| | | | | |
| | Lösung B | | | |
| | Glukose | 5,0 g | | |
| | Wasser | ad 100,0 ml | | |
| | Gebrauchsfertige Lösung herstellen durch Mischen von Lösung A und B | | | |

EP 0 219 784 B1

| Beisp. Nr. | Formulierung | | Mikromol | R |
|---|---|---|---|---|
| 41 | **Lösung A** Lösung A aus Beispiel 72 | | | |
| | **Lösung B** Kochsalz Wasser Gebrauchsfertige Lösung herstellen durch Mischen von Lösung A und B | 2,25 g ad 250 ml | | |
| 42 | **Lösung A** Ciprofloxacinlaktat · 2 H₂O | 276 mg | 604 | 1.00 (darin enthalten equimolare Menge Milchsäure) |
| | Milchsäure 10% (g/g) 0,1 m Salzsäurelösung Wasser | 20 mg 2,00 ml ad 4,0 ml | 22 200 | 0.037 0.33 |
| | **Lösung B** Mannit Wasser Gebrauchsfertige Lösung herstellen durch Mischen von Lösung A und B | 5,0 g ad 100,0 ml | | |
| 43 | **Lösung A** Lösung A aus Beispiel 74 | | | |
| | **Lösung B** Glukose Wasser Gebrauchsfertige Lösung herstellen durch Mischen von Lösung A und B | 25,0 g ad 250,0 ml | | |
| 44 | **Lösung A** Lösung A aus Beispiel 74 | | | |
| | **Lösung B** Kochsalz Wasser Gebrauchsfertige Lösung herstellen durch Mischen von Lösung A und B | 4,5 g ad 500,0 ml | | |
| 45 | **Lösung A** Ciprofloxacin Milchsäure 20% (g/g) Wasser | 100 mg 168 mg ad 1,00 ml | 302 373 | 1.00 1.24 |
| | **Lösung B** Kochsalz Wasser Gebrauchsfertige Lösung herstellen durch Mischen von Lösung A und B | 0,45 g ad 50,0 ml | | |
| 46 | **Lösung A** Lösung A aus Beispiel 77 | | | |
| | **Lösung B** Ringer Laktat-Lösung: Gebrauchsfertige Lösung herstellen durch Mischen von Lösung A und B | 100 ml | (z.B. Ringer Laktat DAB 7 der Fa. Braun Melsungen Rote Liste 1985 Nr. 51013) | |

13

| Beisp. Nr. | Formulierung | | Mikromol | R |
|---|---|---|---|---|
| 47 | Lösung A | | | |
| | Ciprofloxacin | 200 mg | 604 | 1.00 |
| | Milchsäure 25% (g/g) | 257 mg | 715 | 1.18 |
| | Wasser | ad 1,00 ml | | |
| | | | | |
| | Lösung B | | | |
| | Kochsalz | 0,9 g | | |
| | Wasser | ad 100,0 ml | | |
| | Gebrauchsfertige Lösung herstellen durch Mischen von Lösung A und B | | | |
| 48 | Lösung A | | | |
| | Ciprofloxacin | 175 mg | 528 | 1.00 |
| | Milchsäure 20% (g/g) | 300 mg | 667 | 1.26 |
| | Wasser | ad 0,5 ml | | |
| | | | | |
| | Lösung B | | | |
| | Glukose | 2,5 g | | |
| | Wasser | ad 50,0 ml | | |
| | Gebrauchsfertige Lösung herstellen durch Mischen von Lösung A und B | | | |
| 49 | Lösung A | | | |
| | Ciprofloxacin | 200 mg | 604 | 1.00 |
| | Milchsäure 20% (g/g) | 322 mg | 715 | 1.18 |
| | Salzsäure | 3,3 mg | 90 | 0.15 |
| | Kochsalz | 0,9 g | | |
| | Wasser | ad 100,0 ml | | |
| | | | | |
| | Lösung B | | | |
| | Lösung zur Zufuhr von Aminosäuren: | 500 ml | (z.B. Aminoplasmal LS-5 electrolytfrei der Fa. Braun Melsungen Rote Liste 1985 Nr. 51238) | |
| | Gebrauchsfertige Lösung herstellen durch Mischen von Lösung A und B | | | |
| 50 | Lösung A | | | |
| | Ciprofloxacin | 100 mg | 302 | 1.00 |
| | Milchsäure 2% (g/g) | 1481 mg | 329 | 1.09 |
| | Phosphorsäure | 16 mg | 165 | 0.50 |
| | Wasser | ad 10,0 ml pH: 3,8 | | |
| | | | | |
| | Lösung B | | | |
| | Kochsalz | 3,6 g | | |
| | Wasser | ad 400 ml | | |
| | Gebrauchsfertige Lösung herstellen durch Mischen von Lösung A und B | | | |
| 51 | Lyophilisat | | | |
| | Ciprofloxacin | 100 mg | 302 | 1.00 |
| | | | | |
| | Lösung | | | |
| | Milchsäure 10% (g/g) | 322 mg | 358 | 1.18 |
| | Salzsäure | 1,6 mg | 45 | 0.15 |
| | Kochsalz | 900 mg | | |
| | Wasser | ad 100,0 ml | | |
| | Gebrauchsfertige Lösung herstellen durch Auflösen des Lyophilisats in der Lösung | | | |

| Beisp. Nr. | Formulierung | | Mikromol | R |
|---|---|---|---|---|
| 52 | Lyophilisat | | | |
| | Ciprofloxacinlaktat | 63,6 mg | 151 | 1.00 (darin enthalten equimolare Menge Milchsäure) |
| | Lösung | | | |
| | Milchsäure 0,005% (g/g) | 10,87 g | 6 | 1.04 |
| | Kochsalz | 225 mg | | |
| | Wasser | ad 25 ml | | |
| | Gebrauchsfertige Lösung herstellen durch Auflösen des Lyophilisats in der Lösung | | | |
| 53 | Lyophilisat | | | |
| | Ciprofloxacinlaktat | 127 mg | 302 | 1.00 (darin enthalten equimolare Menge Milchsäure) |
| | Milchsäure | 1,1 mg | 12 | 0.04 |
| | Mannit | 5,0 g | | |
| | Lösung | | | |
| | Wasser | ad 100,0 ml | | |
| | Gebrauchsfertige Lösung herstellen durch Auflösen des Lyophilisats in der Lösung | | | |
| 54 | Lyophilisat | | | |
| | Ciprofloxacin | 100 mg | 302 | 1.00 |
| | Lösung | | | |
| | Milchsäure 20% (g/g) | 186 mg | 413 | 1.37 |
| | 0,1 m Salzsäurelösung | 0,42 ml | 142 | 0.47 |
| | Glukose | 25,0 g | | |
| | Wasser | ad 500,0 ml | | |
| | Gebrauchsfertige Lösung herstellen durch Auflösen des Lyophilisats in der Lösung | | | |
| 55 | Lyophilisat | | | |
| | Ciprofloxacinlaktat | 222 mg | 604 | 1.00 (darin enthalten equimolare Menge Milchsäure) |
| | Glukose | 12,5 g | | |
| | Lösung | | | |
| | Milchsäure 20% (g/g) | 100 mg | 222 | 0.37 |
| | Salzsäure | 10,4 mg | 284 | 0.47 |
| | Wasser | ad 250 ml | | |
| | Gebrauchsfertige Lösung herstellen durch Auflösen des Lyophilisats in der Lösung | | | |
| 56 | Lyophilisat | | | |
| | Ciprofloxacinlaktat | 222 mg | 604 | 1.00 (darin enthalten equimolare Menge Milchsäure) |
| | Milchsäure | 10 mg | 111 | 0.18 |
| | Mannit | 5,0 g | | |
| | Lösung | | | |
| | Wasser | 100,0 ml | | |
| | Gebrauchsfertige Lösung herstellen durch Auflösen des Lyophilisats in der Lösung | | | |

| Beisp. Nr. | Formulierung | Mikromol | R |
|---|---|---|---|
| 57 | Lyophilisat Lyophilisat aus Beispiel 92 | | |
| | Lösung Wasser                   200,0 ml Gebrauchsfertige Lösung herstellen durch Auflösen des Lyophilisats in der Lösung | | |

**Patentansprüche**

1. Infusionslösungen der 1-Cyclopropyl-6-fluor-1,4-dihydro-4-oxo-7-(1-piperazinyl)-chinolin-3-carbonsäure (=Ciprofloxacin), enthaltend 0,015 bis 0,5 g des Wirkstoffs auf 100 ml wäßrige Lösung und je nach Wirkstoffkonzentration 1,04 bis 2,20 Mol, bezogen auf 1 Mol Wirkstoff, einer oder mehrerer physiologisch verträglicher Säuren, wobei für den Fall der Anwesenheit mehrerer Säuren deren Gesamtgehalt die Menge von 2,20 Mol, bezogen auf 1 Mol Wirkstoff, nicht übersteigt.

2. Infusionslösungen nach Anspruch 1, enthaltend 0,015 bis 0,5 g des Wirkstoffs auf 100 ml wäßrige Lösung und eine zur Lösung des Wirkstoffs und zur Stabilisierung der Lösung ausreichende Menge einer oder mehrerer Säure(n) aus der Gruppe bestehend aus Salzsäure, Methansulfonsäure, Propionsäure, Bernsteinsäure, Glutarsäure, Zitronensäure, Fumarsäure, Maleinsäure, Weinsäure, Glutaminsäure, Glukonsäure, Glukuronsäure, Galakturonsäure, Ascorbinsäure, Phosphorsäure, Salpetersäure, Essigsäure, Apfelsäure, L-Asparaginsäure und Milchsäure, wobei Milchsäure und Salzsäure bzw. Gemische von Milchsäure und Salzsäure bevorzugt sind.

3. Infusionslösung nach Anspruch 2, enthaltend 0,015 bis 0,5 g des Wirkstoffs und 1,04 bis 1,40 Mol, bezogen auf 1 Mol Wirkstoff, Milchsäure.

4. Infusionslösungen nach Anspruch 2, enthaltend 0,015 bis 0,5 g des Wirkstoffs auf 100 ml wäßrige Lösung und 1,12 bis 1,24 Mol, bezogen auf 1 Mol Wirkstoff, Milchsäure.

5. Infusionslösungen nach Anspruch 2, enthaltend 0,015 bis 0,5 g des Wirkstoffs auf 100 ml wäßrige Lösung und bis zu 5 Mol, bezogen auf 1 Mol Wirkstoff, Milchsäure sowie eine weitere physiologisch verträgliche Säure mit der Maßgabe, daß die gesamte Säuremenge je nach Wirkstoffkonzentration mehr als 1,04 Mol beträgt, jedoch 2,20 Mol, bezogen auf 1 Mol Wirkstoff, nicht übersteigt.

6. Infusionslösungen nach den Ansprüchen 1 bis 5, dadurch gekennzeichnet, daß sie Verdickungsmittel, Resorptionsmittel, Lichtschutzmittel, Resorptionshemmer, Kristallisationsbeschleuniger, Resorptionsbeschleuniger, Kristallisationsverzögerer, Komplexierungsmittel, Antioxidantia, Isotonisierungsmittel und/oder Euhydrierungsmittel als Formulierungshilfsmittel enthalten.

7. Infusionslösungen nach den Ansprüchen 1 bis 6 mit einer Osmolalität von 0,20 bis 0,70 Osm/kg, bevorzugt 0,26 bis 0,39 Osm/kg, enthaltend Isotonisierungsmittel wie NaCl, Sorbit, Mannit, Glukose, Saccharose, Xylit, Fruktose und Glycerin oder Gemische solcher Substanzen und/oder gegebenenfalls Substanzen, die als Bestandteile in gängigen Infusionsträgerlösungen enthalten sind.

8. Infusionslösungen nach den Ansprüchen 1 bis 7, deren pH-Wert 3,0 bis 5,2 beträgt.

9. Infusionslösungen nach den Ansprüchen 1 bis 7, deren pH-Wert 3,6 bis 4,7, insbesondere 3,9 bis 4,5, beträgt.

10. Infusionslösungen nach den Ansprüchen 1 bis 7, deren pH-Wert 4,1 bis 4,3 beträgt.

11. Infusionslösungen nach Anspruch 2, dadurch gekennzeichnet, daß sie außer Wasser, Wirkstoff und sonstigen Formulierungshilfsmitteln eine derartige Menge Kochsalz oder anderer zur Isotonisierung üblicher Hilfsstoffe enthalten, daß eine mit der Gewebeflüssigkeit des menschlichen oder tierischen Körpers isotone oder geringfügig hypo- oder hypertone Lösung vorliegt.

12. Infusionslösungen nach Anspruch 1, dadurch gekennzeichnet, daß sie außer Wirkstoff, Wasser und sonstigen Formulierungshilfsmitteln 1,04 bis 1,40 Mol Milchsäure und 0,0 bis 0,80 Mol Salzsäure (jeweils bezogen auf 1 Mol Wirkstoff) und bezogen auf 100 ml Lösung 0,6 bis 2,2 g NaCl, vorzugsweise 0,75 bis 1,20 g, insbesondere 0,85 bis 0,95 g NaCl enthalten.

13. Infusionslösungen nach den Ansprüchen 1 bis 12 in zur Infusion geeigneten Dosierungseinheiten, die sowohl aus Glas oder aus dazu geeigneten Kunststoffen hergestellt sein können, mit entnehmbaren Inhalten von 40 bis 600 ml, vorzugsweise von 50 bis 120 ml.

14. Lyophilisate, erhalten durch einen Gefriertrocknungsprozeß, sowie Konzentrate oder sonstige Darreichungsformen, die vor der Applikation in Infusionslösungen nach den Ansprüchen 1 bis 16 überführt werden können.

15. Verfahren zur Herstellung von Infusionslösungen, enthaltend 0,015 bis 0,5 Gew.-% 1-Cyclopropyl-6-fluor-1,4-dihydro-4-oxo-7-(1-piperazinyl)-chinolin-3-carbonsäure (=Ciprofloxacin), dadurch gekennzeichnet, daß man eine geeignete Menge des Wirkstoffs, gegebenenfalls in Form eines Salzes wie Alkali- oder Erdalkalisalzes oder Additionssalzes, eines Hydrates oder eines Hydrates des Salzes oder in Form von Mischungen dieser Salze bzw. Hydrate, mit der Menge einer physiologisch verträglichen Säure

16

versetzt oder eines Gemisches mehrerer physiologisch verträglicher Säuren, die in bezug auf die eben zur Auflösung des Wirkstoffs bzw. seiner Salze oder Hydrate ausreichende Menge einen Ausscheidungen des Wirkstoffs verhindernden Überschuß darstellt, gegebenenfalls Formulierungshilfsmittel hinzufügt und mit Wasser oder einer üblichen Infusionsträgerlösung derart auffüllt, daß sich ein Konzentrationsbereich von 0,015 bis 0,5 g für den Wirkstoff einstellt, wobei beim Einsatz eines Alkali- oder Erdalkalisalzes des Wirkstoffs in den zur Auflösung erforderlichen Säuremengen zusätzlich noch die Mengen enthalten sind, die zur Neutralisation des Wirkstoffanions notwendig sind und beim Einsatz von Additionssalzen ein Teil der erforderlichen Säuremengen bereits im einzusetzenden Wirkstoffsalz enthalten ist.

16. Verfahren nach Anspruch 15, dadurch gekennzeichnet, daß man für den Fall der Verwendung des Wirkstoffs in Salzform eine Säure verwendet, deren Anion dem Anion des Wirkstoffsalzes bzw. des -salzhydrates entspricht.

17. Verfahren nach Anspruch 15, dadurch gekennzeichnet, daß man den Wirkstoff gegebenenfalls in Wasser suspendiert, mit bis zu 5 Mol, bezogen auf 1 Mol Wirkstoff, Milchsäure versetzt und dann gegebenenfalls eine weitere physiologisch verträgliche Säure oder ein Gemisch solcher Säuren, insbesondere Salzsäure, hinzufügt mit der Maßgabe, daß die Gesamtmenge an Säure 2,20 Mol, bezogen auf 1 Mol Wirkstoff, nicht überschreitet, jedoch 1,04 Mol, bezogen auf 1 Mol Wirkstoff, übersteigt und anschließend gegebenenfalls weitere Formulierungshilfsmittel, insbesondere NaCl, das gegebenenfalls auch durch eine Neutralisationsreaktion im Formulierungsansatz entsteht, hinzufügt und mit Wasser zur Einstellung der gewünschten Wirkstoffkonzentration auffüllt.

18. Verfahren nach den Ansprüchen 15 bis 17, dadurch gekennzeichnet, daß man mit (physiologisch) verträglichen Puffersystemen den pH-Wert der Infusionslösungen auf 3,0 bis 5,2, insbesondere 3,6 bis 4,7, einstellt.

19. Verfahren nach den Ansprüchen 15 bis 18, dadurch gekennzeichnet, daß die Herstellung der Infusionslösungen durch Erwärmen erfolgt.

20. Verwendung von Infusionslösungen und/oder sonstigen Darreichungsformen, die vor der Applikation in die Infusionslösungen nach den Ansprüchen 1 bis 14 überführt werden, zur Herstellung von zur Infusion geeigneten Dosierungseinheiten mit entnehmbaren Inhalten von 40 bis 600 ml.

**Revendications**

1. Solutions pour infusion au perfusion de l'acide 1-cyclopropyl-6-fluoro-1,4-dihydro-4-oxo-7-(1-pipérazinyl)-quinoléine-3-carboxylique (=ciprofloxacine), contenant 0,015 à 0,5 g de la substance active pour 100 ml der solution aqueuse et, selon la concentration en la substance active, 1,04 à 2,20 moles, pour 1 mole de la substance active, d'un ou plusieurs acides physiologiquement tolérables, et, en cas de présence de plusieurs acides, leur teneur totale n'excède pas la quantité de 2,20 moles pour 1 mole de la substance active.

2. Solutions pour infusion ou perfusion selon la revendication 1, contenant 0,015 à 0,5 g de la substance active pour 100 ml de la solution aqueuse et une quantité, suffisante pour dissoudre la substance active et pour stabiliser la solution, d'un ou plusieurs acides choisis dans l'ensemble consistant en l'acide chlorhydrique, l'acide méthanesulfonique, l'acide propionique, l'acide succinique, l'acide glutarique, l'acide citrique, l'acide fumarique, l'acide maléique, l'acide tartrique, l'acide glutamique, l'acide gluconique, l'acide glucuronique, l'acide galacturonique, l'acide ascorbique, l'acide phosphorique, l'acide nitrique, l'acide acétique, l'acide malique, l'acide L-asparagique et l'acide lactique, parmi lesquels on préfère l'acide lactique et l'acide chlorhydrique ou bien des mélanges de l'acide lactique et de l'acide chlorhydrique.

3. Solutions pour infusion ou perfusion selon la revendication 2, contenant 0,015 à 0,5 g de la substance active et 1,04 à 1,40 mole, pour 1 mole de la substance active, d'acide lactique.

4. Solutions pour infusion ou perfusion selon la revendication 2, contenant 0,015 à 0,5 g de la substance active pour 100 ml de la solution aqueuse et 1,12 à 1,24 mole d'acide lactique, pour 1 mole de la substance active.

5. Solutions pour infusion ou perfusion selon la revendication 2, contenant 0,015 à 0,5 g de la substance active pour 100 ml de solution aqueuse et jusqu'à 5 moles, pour 1 mole de la substance active, d'acide lactique ainsi qu'un autre acide physiologiquement tolérable, à la condition que, selon la concentration en la substance active, la quantité totale des acides soit supérieure à 1,04 mole mais n'excède pas 2,20 moles, par 1 mole de la substance active.

6. Solutions pour infusion ou perfusion selon les revendications 1 à 5, caractérisées en ce qu'elles contiennent des épaississants, des agents de résorption, des agents de protection contre les effets de la lumière, des inhibiteurs de résorption, des accélérateurs de cristallisation, des accélérateurs de la résorption, des agents retardant une cristallisation, des agents de complexation, des antioxydants, des agents d'isotonisation et/ou des agents d'hydrogénation ou d'hydratation, comme adjuvants de formulation.

7. Solutions pour infusion ou perfusion selon les revendications 1 à 6, ayant une osmolalité de 0,20 à 0,70 osm/kg, de préférence 0,26 à 0,39 osm/kg, contenant un agent rendant isotonique (agent d'isotonisation) comme NaCl, le sorbitol, le mannitol, le glucose, le saccharose, le xylitol, le fructose et le glycérol ou des mélanges de ces substances et/ou éventuellement des substances contenues comme parties constitutives de solutions usuelles dans un véhicule pour infusion.

8. Solutions pour infusion ou perfusion selon les revendications 1 à 7, dont le pH vaut de 3,0 à 5,2.

9. Solutions pour infusion ou perfusion selon les revendications 1 à 7, dont le pH vaut de 3,6 à 4,7, en particulier 3,9 à 4,5.

10. Solutions pour infusion ou perfusion selon les revendications 1 à 7, dont le pH vaut de 4,1 à 4,3.

11. Solutions pour infusion ou perfusion selon la revendication 2, caractérisées en ce qu'en plus de l'eau, de la substance active et d'autres adjuvants de formulation, les solutions contiennent une quantité de sel de cuisine, ou d'autres adjuvants usuels pour une isotonisation, telle que l'on obtienne une solution isotonique par rapport aux liquides des tissus du corps humain ou animal, ou une solution légèrement hypotonique ou hypertonique.

12. Solutions pour infusion ou perfusion selon la revendication 1, caractérisées en ce qu'en plus de la substance active, de l'eau et d'autres adjuvants de formulation, les solutions contiennent 1,04 à 1,40 mole d'acide lactique et 0,0 à 0,80 mole d'acide chlorhydrique (proportion rapportée à chaque fois à 1 mole de la substance active) et, pour 100 ml de solution, 0,6 à 2,2 g de NaCl, avantageusement 0,75 à 1,20 g et en particulier 0,85 à 0,95 g de NaCl.

13. Solutions pour infusion ou perfusion selon les revendications 1 à 12, en des unités dosées convenant pour une infusion, pouvant être préparées en du verre ou en des matières plastiques convenant à cet effet, et dont le contenu prélevable est de 40 à 600 ml, avantageusement de 50 à 120 ml.

14. Lyophilisats obtenus par un procédé de lyophilisation, ainsi que des concentrés et d'autres formes de présentation qui peuvent, avant l'administration, être transformés en des solutions pour infusion ou perfusion selon les revendications 1 à 13.

15. Procédé pour préparer des solutions pour infusion ou perfusion, contenant 0,015 à 0,5% en poids d'acide 1-cyclopropyl-6-fluoro-1,4-dihydro-4-oxo-7-(1-pipérazinyl)-quinoléine-3-carboxylique (=ciprofloxacine), procédé caractérisé en ce qu'on ajoute à une quantité convenable de la substance active, éventuellement sous forme d'un sel tel qu'un sel alcalin ou alcalino-terreux ou un sel d'addition, un hydrate ou un hydrate du sel ou sous forme de mélange de ces sels ou hydrates, la quantité d'un acide physiologiquement tolérable ou d'un mélange de plusieurs acides physiologiquement tolérables qui représente, par rapport à la quantité suffisant juste pour dissoudre la substance active ou son sel ou ses hydrates, un excès destiné à empêcher une séparation ou un dépôt de la substance active, on ajoute éventuellement des adjuvants de formulation et l'on complète avec de l'eau ou avec une solution usuelle constituant un véhicule de support d'infusion de manière à ajuster la concentration de la substance active dans un domaine compris entre 0,015 et 0,5 g et, en cas d'utilisation d'un sel alcalin ou alcalino-terreux de la substance active, la solution contient en outre les quantités nécessaires pour la neutralisation de l'anion de la substance active et, en cas d'utilisation de sels d'addition, une partie de la quantité d'acide nécessaire est déjà contenue dans le sel de substance active à utiliser.

16. Procédé selon la revendication 15, caractérisé en ce qu'en cas d'utilisation de la substance active sous forme d'un sel, on utilise un acide dont l'anion correspond à l'anion du sel de la substance active ou de l'hydrate de sel de la substance active.

17. Procédé selon la revendication 15, caractérisé en ce qu'on met la substance active en suspension éventuellement dans de l'eau, en y ajoute jusqu'à 5 moles, pour 1 mole de la substance active, d'acide lactique puis l'on introduit éventuellement un autre acide physiologiquement tolérable ou un mélange de tels acides, en particulier l'acide chlorhydrique, à la condition que la quantité totale de l'acide n'excède pas 2,20 moles, pour 1 mole de la substance active, mais excède 1,04 mole, pour 1 mole de la substance active, et l'on introduit ensuite éventuellement d'autres adjuvants de formulation, en particulier NaCl, qui résulte éventuellement aussi d'une réaction de neutralisation se déroulant dans la charge de la formulation, et l'on complète avec de l'eau pour obtenir la concentration voulue en la substance active.

18. Procédé selon les revendications 15 à 17, caractérisé en ce qu'à l'aide de systèmes de tampon (physiologiquement) tolérables, on ajuste le pH des solutions pour infusion ou perfusion à une valeur de 3,0 à 5,2 notamment 3,6 à 4,7.

19. Procédé selon les revendications 15 à 18, caractérisé en ce que la préparation des solutions pour infusion ou perfusion à lieu par chauffage.

20. Utilisation de solutions pour infusion ou perfusion et/ou d'autres formes de présentation qui, avant l'administration, ont été transformées en les solutions pour infusion ou perfusion selon les revendications 1 à 14, pour préparer des unités dosées convenant pour une infusion ou perfusion et comportant des contenus prélevables de 40 à 600 ml.

**Claims**

1. Infusion solutions of 1-cyclopropyl-6-fluoro-1,4-dihydro-4-oxo-7-(1-piperazinyl)-quinoline-3-carboxylic acid (=ciprofloxacin) containing 0.015 to 0.5 g of the active compound per 100 ml of aqueous solution and, depending on the active compound concentration, 1.04 to 2.20 mol, relative to 1 mol of active compound, of one or more physiologically tolerated acies, and where when several acids are present their total content does not exceed the amount of 2.20 mol, relative to 1 mol of active compound.

2. Infusion solutions according to Claim 1, containing 0.015 to 0.5 g of the active compound per 100 ml of aqueous solution and an amount, which suffices to dissolve the active compound and to stabilize the solution, of one or more acid(s) from the group comprising hydrochloric acid, methanesulphonic acid, propionic acid, succinic acid, glutaric acid, citric acid, fumaric acid, maleic acid, tartaric acid, glutamic acid, gluconic acid, glucuronic acid, galacturonic acid, ascorbic acid, phosphoric acid, nitric acid, acetic acid, malic acid, L-aspartic acid and lactic acid, preference being given to lactic acid and hydrochloric acid or mixtures of lactic acid and hydrochloric acid.

3. Infusion solution according to Claim 2, containing 0.015 to 0.5 g of the active compound and 1.04 to 1.40 mol, relative to 1 mol of active compound, of lactic acid.

4. Infusion solutions according to Claim 2, containing 0.015 to 0.5 g of the active compound per 100 ml of aqueous solution and 1.12 to 1.24 mol, relative to 1 mol of active compound, of lactic acid.

5. Infusion solutions according to Claim 2, containing 0.015 to 0.5 g of the active compound per 100 ml of aqueous solution and up to 5 mol, relative to 1 mol of active compound, of lactic acid and another physiologically tolerated acid with the proviso that the total amount of acid is, depending on the active compound concentration, more than 1.04 mol but does not exceed 2.20 mol, relative to 1 mol of active compound.

6. Infusion solutions according to Claims 1 to 5, characterized in that they contain thickeners, resorbents, light protection agents, absorption inhibitors, crystallization accelerators, absorption accelerators, crystallization retardants, complexing agents, antioxidants, isotonicizing agents and/or euhydrogenation agents as formulating auxiliaires.

7. Infusion solutions according to Claims 1 to 6 having an osmolality of 0.20 to 0.70 Osm/kg, preferably 0.26 to 0.39 Osm/kg, containing isotonicizing agents such as NaCl, sorbitol, mannitol, glucose, sucrose, xylitol, fructose and glycerol or mixtures of such substances and/or, where appropriate, substances which are contained as constituents in conventional infusion vehicle solutions.

8. Infusion solutions according to Claims 1 to 7, whose pH is 3.0 to 5.2.

9. Infusion solutions according to Claims 1 to 7 whose pH is 3.6 to 4.7, in particular 3.9 to 4.5.

10. Infusion solutions according to Claims 1 to 7, whose pH is 4.1 to 4.3.

11. Infusion solutions according to Claim 2, characterized in that, in addition to water, active compound and other formulating auxiliaries, they contain an amount of sodium chloride, or other auxiliaires customary for isotonicizing, such that the solution is in a form which is isotonic, or slightly hypo- or hypertonic, with the tissue fluid in the human or animal body.

12. Infusion solutions according to Claim 1, characterized in that, in addition to active compound, water and other formulating auxiliaries, they contain 1.04 to 1.40 mol of lactic acid and 0.0 to 0.80 mol of hydrochloric acid (in each case relative to 1 mol of active compound), and relative to 100 ml of solution, 0.6 to 2.2 g of NaCl, preferably 0.75 to 1.20 g, in particular 0.85 to 0.95 g of NaCl.

13. Infusion solutions according to Claims 1 to 12 in dosage units which are suitable for infusion and which can be prepared both from glass or from plastics suitable for this purpose, having removable contents of 40 to 600 ml, preferably from 50 to 120 ml.

14. Lyophilizates obtained by a freeze-drying process, and concentrates or other presentations which, before administration, can be converted into infusion solutions according to Claims 1 to 13.

15. Process for the preparation of infusion solutions, containing 0.015 to 0.5% by weight of 1-cyclopropyl-6-fluoro-1,4-dihydro-4-oxo-7-(1-piperazinyl)-quinoline-3-carboxylic acid (=ciprofloxacin), characterized in that a suitable amount of the active compound, where appropriate in the form of a salt, such as of an alkali metal or alkaline earth metal salt or addition salt, of a hydrate or of a hydrate of the salt, or in the form of mixtures of these salts or hydrates, is mixed with the amount of a physiologically tolerated acid, or of a mixture of several physiologically tolerated acids which, in relation to the amount which just suffices to dissolve the active compound or its salts or hydrates, represents an excess preventing separation out of the active compound, formulating auxiliaries are added where appropriate, and the mixture is made up with water or a customary infusion vehicle solution in such a manner that the concentration of the active compound is adjusted to the range from 0.015 to 0.5 g, and when an alkali or alkaline earth metal salt of the active compound is used the amounts of acid necessary for the dissolution additionally contain the amounts which are needed to neutralize the active compound anion, and when addition salts are used a part of the amounts of acid which are necessary is already contained in the active compound salt to be used.

16. Process according to Claim 15, characterized in that, in the case where the active compound is used in a salt form, use is made of an acid whose anion corresponds to the anion of the active compound salt or salt hydrate.

17. Process according to Claim 15, characterized in that the active compound is, where appropriate, suspended in water, and up to 5 mol, relative to 1 mol of active compound, of lactic acid are added, and then, where appropriate, another physiologically tolerated acid or a mixture of such acids, in particular hydrochloric acid, is added, with the proviso that the total amount of acid does not exceed 2.20 mol, relative to 1 mol of active compound, but does exceed 1.04 mol, relative to 1 mol of active compound, and then, where appropriate, further formulating auxiliaries are added, in particular NaCl, which is also, where appropriate, produced by a neutralization reaction in the formulation mixture, and the desired active compound concentration is adjusted by making up with water.

18. Process according to Claims 15 to 17, characterized in that the pH of the infusion solutions is adjusted to 3.0 to 5.2, in particular 3.6 to 4.7, with (physiologically) tolerated buffer systems.

19. Process according to Claims 15 to 18, characterized in that the infusion solutions are prepared by heating.

20. Use of infusion solutions and/or other presentations which, before administration, are converted into the infusion solutions according to Claims 1 to 14 for the preparation of dosage units which are suitable for infusion and have removable contents of 40 to 600 ml.